# EUROPEAN PATENT SPECIFICATION

(11) **EP 4 091 608 B1**
(45) Date of publication and mention of the grant of the patent: **17.04.2024**
(21) Application number: 21175228.2
(22) Date of filing: 21.05.2021
(51) Int. Cl.: A61K 31/14, A61K 31/245, A61K 38/12, A61P 31/12, A61P 31/14, A61P 31/16

(54) **COMPOSITION WITH ANTIVIRAL EFFECT**
ZUSAMMENSETZUNG MIT ANTIVIRALER WIRKUNG
COMPOSITION AVEC EFFET ANTIVIRAL

(43) Date of publication of application: 23.11.2022
(73) Proprietor: Medice Arzneimittel Pütter GmbH & Co. KG, 58638 Iserlohn (DE)
(72) Inventor: AMMER, Richard, 58644 Iserlohn (DE)
(74) Representative: Isarpatent

(56) References cited:
- WO-A1-2017/046312
- Michael Schmidbauer: "Pharmazeutische Zeitung online: In-vitro-Untersuchung: Dorithricin wirkt antiviral", , 16 September 2015 (2015-09-16), pages 1-4, XP055317657, Retrieved from the Internet: URL:http://www.pharmazeutische-zeitung.de/ index.php?id=59741 [retrieved on 2016-11-09]
- Anonymous: "Neue wissenschaftliche Studie zeigt: Dorithricin kann SARS-CoV-2 inaktivieren", , 25 August 2021 (2021-08-25), XP055855116, Retrieved from the Internet: URL:https://www.dorithricin.de/www/PDF_Stu die_Dorithricin_2021-08-25.pdf [retrieved on 2021-10-26]

## Description

### Field of the invention

The present invention concerns a pharmaceutical composition comprising tyrothricin, benzalkonium chloride and benzocaine for use in anti-viral therapy or in the treatment of a viral infection, wherein the viral infection is caused by coronaviruses.

### Background of the Invention

Viruses and bacteria are the pathogens of the very common acute pharyngitis. For the treatment of these diseases, drugs have been available on the market for decades, such as Dorithricin^{®} throat lozenges Classic. These are usually used with the antiseptic and antibacterial agents contained therein for the clinically effective relief of the typical pharyngitis symptoms such as swallowing pain and redness of the pharyngitis.

Tyrothricin is a mixture of various anti-bacterially effective linear and cyclic polypeptides from the groups of gramicidins and tyrocidins. They are formed endotoxin-like by the anaerobic spore-forming Bacillus aneurinolyticus (Syn. Bacillus brevis). The area of activity includes mainly gram-positive bacteria, but also some gram-negative bacteria and various types of fungi, such as Candida albicans. Tyrothricin contains 50 to 70% tyrocidins and 25 to 50% gramicidins, which together make up at least 85% of the active ingredient. In addition, small quantities of other structurally related polypeptides occur.

Benzalkonium chloride is a mixture of alkylbenzyldimethylammonium chlorides whose alkyl moiety consists of C8 to C18 chains. It has a general disinfecting and conservative effect and is effective against bacteria, fungi, yeasts and algae (even antiviral to a lesser extent).

Benzocaine (4-aminobenzoic acid ethyl ester) is a local anaesthetic and is mainly used for localized pain therapy of the skin and mucous membrane, such as in the mouth and throat, as well as in medicines against colds, cough-killing preparations, painkillers such as solutions or lozenges for throat, stomach and tooth problems, astringents.

WO 2017/046312 discloses a pharmaceutical composition with antiviral effect.

### Summary of the invention

The present invention concerns a pharmaceutical composition comprising tyrothricin, benzalkonium chloride and benzocaine for use in anti-viral therapy or in the treatment of a viral infection, wherein the viral infection is caused by coronaviruses.

Further aspects and embodiments of the invention are disclosed in the dependent claims and can be taken from the following description, figures and examples, without being limited thereto.

### Figures

The enclosed drawings should illustrate embodiments of the present invention and convey a further understanding thereof. In connection with the description, they serve as explanation of concepts and principles of the invention. Other embodiments and many of the stated advantages can be derived in relation to the drawings. The elements of the drawings are not necessarily to scale towards each other. Identical, functionally equivalent and acting equal features and components are denoted in the figures of the drawings with the same reference numbers, unless noted otherwise.

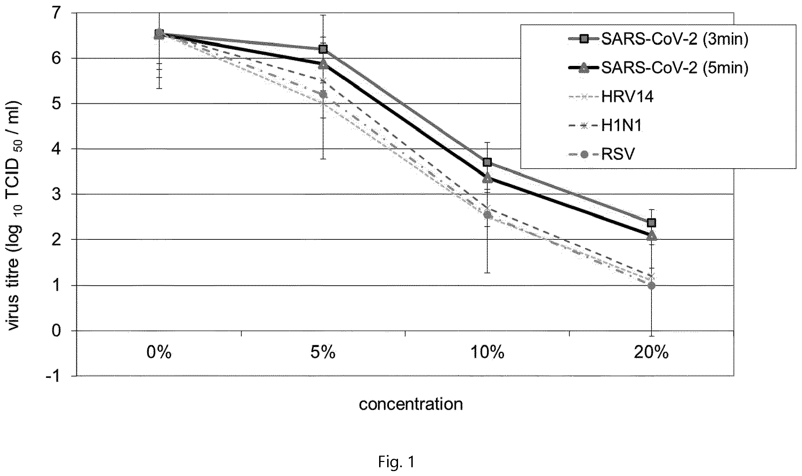

Figure 1: In vitro study of anti-viral efficacy at increasing dilution of tyrothricin, benzalkonium chloride and benzocaine comprised in 1 tablet of Dorithricin^{®} tablets studied in artificial nasal secrete and different viruses (SARS-CoV-2 after 3 minutes of exposure, and SARS-CoV-2, HRV14, H1N1, RSV after 5 minutes of exposure). Antiviral effect of triple combination sore throat lozenge Dorithricin^{®} tablets measured by virus titre in relation to concentration of triple active ingredients (all 3 active ingredients).

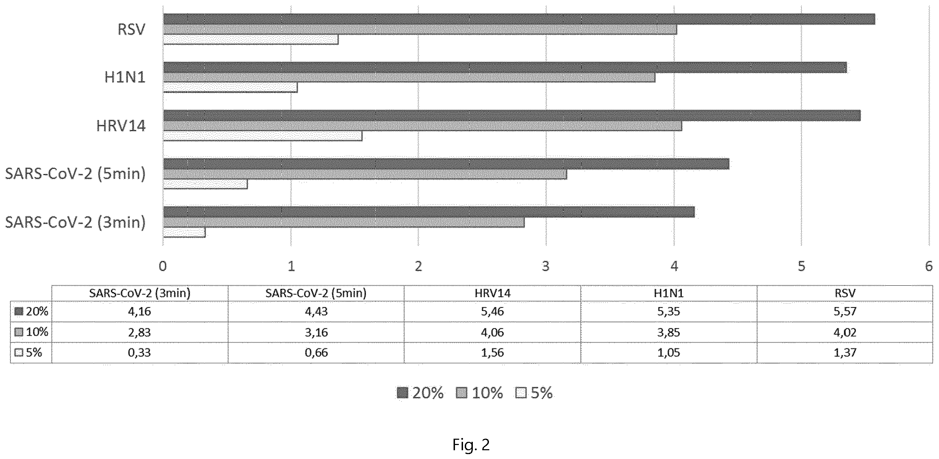

Figure 2: In vitro study of anti-viral efficacy at increasing dilution of tyrothricin, benzalkonium chloride and benzocaine comprised in 1 tablet of Dorithricin^{®} tablets studied in artificial nasal secrete and different viruses (SARS-CoV-2 after 3 minutes of exposure, and SARS-CoV-2, HRV14, H1N1, RSV after 5 minutes of exposure). Antiviral effect of triple combination sore throat lozenge Dorithricin^{®} tablets measured by Reduction Factor in relation to concentration of triple active ingredients (all 3 active ingredients).

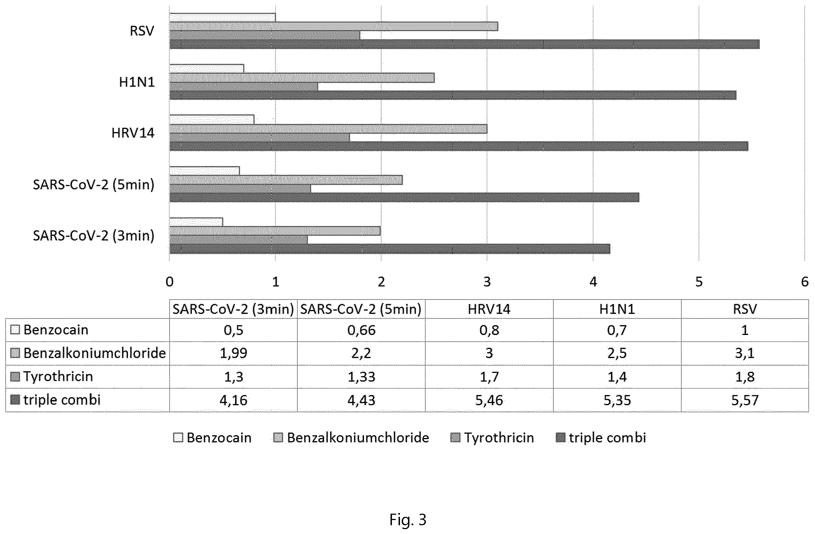

Figure 3: In vitro study of anti-viral efficacy at 20% concentration of tyrothricin, benzalkonium chloride and benzocaine in combination compared to the three compounds applied alone studied on all viruses (RSV, H1N1, HRV14, SARS-CoV-2 after 3 and 5 minutes of exposure). Antiviral effect of triple combination sore throat lozenge Dorithricin^{®} tablets measured by Reduction Factor in relation to concentration of triple active ingredients (all 3 active ingredients separately and combined).

### Detailed description of the present invention

Unless defined otherwise, technical and scientific terms used herein have the same meaning as commonly understood by one of ordinary skill in the art to which this invention belongs.

Amounts within the present invention are given in wt.%, unless stated otherwise or clear from context.

Dorithricin^{®} tablets (MEDICE, Iserlohn, Germany) are tablets containing tyrothricin, benzalkonium chloride and benzocaine. One tablet contains 0.5 mg of tyrothricin, 1.0 mg of benzalkonium chloride, and 1.5 mg of benzocaine.

Before the invention is described in exemplary detail, it is to be understood that this invention is not limited to the particular component parts of the process steps of the methods described herein as such methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. For example, the term "a" as used herein can be understood as one single entity or in the meaning of "one or more" entities. It is also to be understood that plural forms include singular and/or plural referents unless the context clearly dictates otherwise. It is moreover to be understood that, in case parameter ranges are given which are delimited by numeric values, the ranges are deemed to include these limitation values.

In a first aspect, the present invention refers to a pharmaceutical composition comprising tyrothricin, benzalkonium chloride and benzocaine for use in anti-viral therapy or in the treatment of viral infection, wherein the viral infection is caused by coronaviruses.

The in vitro studies of Figures 1-3 demonstrate that there is an over-additive, synergistic anti-viral effect on various viruses (SARS-CoV-2, HRV14, H1N1, RSV), as the combination of tyrothricin, benzalkonium chloride and benzocaine is more potent than the individual substances by a factor of 3 to 11. This super-additive effect including activity against corona viruses is novel, not yet reported in science, and surprising: benzocaine is a sodium channel blocker and known as a local anesthetic (1), benzalkonium chloride is an antiseptic with known antiviral activity (2-5), tyrothricin acts as an antimicrobial peptide predominantly antibacterial on the cell membrane of bacteria (6-8).

The composition of the present invention may include, consist of or substantially comprise the above-mentioned active substances. The formulation "essentially consists of" means that the pharmaceutical composition contains these components as effective ingredients. However, it may still contain one or more solvents, excipients, etc., which are necessary for the manufacture of a pharmaceutical composition, but which do not contribute or do not make a significant contribution to the pharmacological efficacy.

The term "pharmaceutical composition" as used herein includes, in particular, peroral dosage forms such as solid, semi-liquid or liquid compositions for oral administration. In a preferred embodiment, the composition is in the form of a tablet, in particular a lozenge or throat tablet.

Alternatively, however, other dosage forms are also possible, such as solid peroral dosage forms such as powders or capsules. Here, limited additives of magnesium stearate or calcium behenate can be used as lubricants and of starch as decomposition accelerators (disintegrant).

In the case of granulation, aqueous lactose solution, ethanol and suitable concentrations of starch pastes can be used.

Common auxiliaries used in the manufacture of a throat lozenge include sorbitol (E 420), talc, sucrose fatty acid esters, saccharin sodium dihydrate, mint oil, povidone 25, and carmellose sodium.

In addition to peroral dosage forms, the pharmaceutical composition of the present invention may include parenterals, i. e. liquid dilutions for injection and liquid rubs as well as ointments, suppositories, eye drops and nose drops.

The viral infection is caused by coronaviruses. In some embodiments, the viral infection is caused by SARS-CoV-2 Coronavirus.

A single dose of the pharmaceutical composition preferably contains about 0.5 mg of tyrothricin, about 1.0 mg of benzalkonium chloride and/or about 1.5 mg of benzocaine and one or more pharmaceutically acceptable excipients. In this context, the term "about" means a variation range of ± 50%, preferably ± 20%, most preferably ± 10% in relation to the active substance mass given above.

According to some embodiments, the pharmaceutical composition of the present invention is a throat tablet. In certain embodiments, the pharmaceutical composition of the present invention is administered in a daily dose of approximately 2-4 mg tyrothricin, 4-8 mg benzalkonium chloride and 6-12 mg benzocaine. In an exemplary embodiment, the pharmaceutical composition corresponds to the composition of Dorithricin^{®} tablets.

The above embodiments can be combined arbitrarily, if appropriate. Further possible embodiments and implementations of the invention comprise also combinations of features not explicitly mentioned in the foregoing or in the following with regard to the Examples of the invention. Particularly, a person skilled in the art will also add individual aspects as improvements or additions to the respective basic form of the invention.

### Working Example

### 1. Preparation of test virus suspension

For virus production, 2 ×10⁶ *Vera E6 cells* were cultivated in a 75 cm² flask in DMEM supplemented with 1 % L-Glut, NEAAs, and P/S and 10 % FBS. One day after seeding Medium was changed to 10 mL fresh DMEM inoculated with 100 µl of SARS-CoV-2/Germany virus suspension. The supernatant was harvested after 3 days at 37°C by centrifugation at 1,500 rpm for 5 min to remove cell debris. Viral titres were determined by plaque assay and endpoint dilution. The supernatant was aliquoted and stored at -80°C.

### 2. Preparation of disinfectant (dilutions)

The test product was tested as solutions with 20.0 g / 100 ml (20.0 %), 10.0 g / 100 ml (10.0 %) and 5.0 g / 100 ml (5.0 %) (demonstrating of non-active range) Dorithricin^{®} tablets with artificial nasal secrete as interfering substance (1 part test virus suspension + 1 part interfering substance + 8 parts disinfectant). Due to the addition of interfering substance and test virus suspension the solutions had to be prepared by the factor 1.25. For preparation of a solution with 20.0 g / 100 ml, the lozenges ground into a fine powder with a mortar and 25.0 g of this powder were added to 50 ml of WSH and stirred for 20 minutes, filled up to a total volume of 100 ml and afterwards immediately used for the inactivation assay. The further dilutions were prepared analogous.

### 3. Preparation of soil load

For the preparation of the artificial nasal secrete, 25 µl BSA stock solution (0.5 g of BSA in 10 ml phosphate buffer), 100 µl mucin stock solution (0.04 g bovine mucin in 10 ml of phosphate buffer) and 35 µl yeast extract stock solution (0.5 g yeast extract in 10 ml phosphate buffer) were mixed.

In the inactivation assays, one pan of this interfering substance was mixed with 1 part test virus suspension and 8 parts of the test product.

### 4. Calculation and verification of virucidal activity

The virucidal activity of the test disinfectant was evaluated by calculating the decrease in title in comparison with the control titration without disinfectant. The difference is given as reduction factor (RF).

According to the EN 14476, a disinfectant or a disinfectant solution at a particular concentration is having virus-inactivating efficacy if the title is reduced at least by 4 10910 steps within the recommended exposure period. This corresponds to an inactivation of a 99.99 %.

The results of the study are depicted in Figures 1-3 and show an over-additive, synergistic anti-viral effect of the combination of tyrothricin, benzalkonium chloride and benzocaine.

### Literature

1) Kumar M, Chawla R, Goyal M. Topical anesthesia. Journal of Anaesthesiology, Clinical Pharmacology. 2015; 31(4):450-456.
2) Weibel MA, Cortat M, Lebek G, LeCotonnec JY, Kitler ME, Barcherini G. An approach of the in vivo antibacterial activity of benzaconium chloride and comparison with other buccopharyngeal disinfectants. Arzneimittelforschung. 1987; 37(4):467-71.
3) Iwasawa A, Niwano Y, Kohno M, Ayaki M. Virucidal activity of alcohol-based hand rub disinfectants. Biocontrol Sci. 2012; 17(1):45-9.
4) Yamasaki H, Tsujimoto K, Ikeda K, Suzuki Y, Arakawa T, Koyama AH. Antiviral and virucidal activities of nα-cocoyl-L-arginine ethyl ester in comparison to benzalkonium chloride. Adv Virol. 2011; 11(28).
5) Wood A, Payne D. The action of three antiseptics/disinfectants against enveloped and non-enveloped viruses. J Hosp Infect. 1998; 38(4):283-95.
6) Lang C, Staiger C. Tyrothricin-An underrated agent for the treatment of bacterial skin infections and superficial wounds? Pharmazie. 2016; 71(6):299-305.
7) Korting HC, Schöllmann C, Stauss-Grabo M, Schäfer-Korting M. Antimicrobial peptides and skin: a paradigm of translational medicine. Skin Pharmacol Physiol. 2012; 25(6):323-34.
8) Bals R. Antimicrobial peptides and peptide antibiotics. Med Klin. 2000; 95(9):496-502.
9) EN 14476:2013+A2:2019: Chemical disinfectants and antiseptics - Quantitative suspension test for the evaluation of virucidal activity of chemicals disinfectants and antiseptics in human medicine test - Test method and requirements (phase 2, step 1).
10) Spearman, C.: The method of "right or wrong cases" (constant stimuli) without Gauss's formulae. Brit J Psychol; 2 1908, 277-242.
11) Kärber, G.: Beitrag zur kollektiven Behandlung pharmakologischer Reihenversuche. Arch Exp Path Pharmak; 162, 1931, 480-487.

## Claims

1. A pharmaceutical composition comprising tyrothricin, benzalkonium chloride and benzocaine for use in a method of treating a viral infection,
wherein the viral infection is caused by coronaviruses.

2. A pharmaceutical composition for use in a method of treating a viral infection according to claim 1, wherein the viral infection is caused by SARS-CoV-2 Coronavirus.

3. A pharmaceutical composition for use in a method of treating a viral infection according to claim 1 or 2, comprising about 0.5 mg of tyrothricin, about 1.0 mg of benzalkonium chloride and about 1.5 mg of benzocaine as a single dose and one or more pharmaceutically acceptable excipients.

4. A pharmaceutical composition for use in a method of treating a viral infection according to one or more of the preceding claims, which is a throat tablet.

5. A pharmaceutical composition for use in a method of treating a viral infection according to one or more of the preceding claims, wherein the composition is administered in a daily dose of about 2-4 mg tyrothricin, 4-8 mg benzalkonium chloride and 6-12 mg benzocaine combined.

## Patentansprüche

1. Pharmazeutische Zusammensetzung, umfassend Tyrothricin, Benzalkoniumchlorid und Benzocain zur Verwendung in einem Verfahren zur Behandlung einer Virusinfektion, wobei die Virusinfektion durch Coronaviren verursacht wird.

2. Pharmazeutische Zusammensetzung zur Verwendung in einem Verfahren zur Behandlung einer Virusinfektion nach Anspruch 1, wobei die Virusinfektion durch das SARS-CoV-2-Coronavirus verursacht wird.

3. Pharmazeutische Zusammensetzung zur Verwendung in einem Verfahren zur Behandlung einer Virusinfektion nach Anspruch 1 oder 2, umfassend etwa 0,5 mg Tyrothricin, etwa 1,0 mg Benzalkoniumchlorid und etwa 1,5 mg Benzocain als Einzeldosis und einen oder mehrere pharmazeutisch akzeptable Hilfsstoffe.

4. Pharmazeutische Zusammensetzung zur Verwendung in einem Verfahren zur Behandlung einer Virusinfektion nach einem oder mehreren der vorstehenden Ansprüche, bei der es sich um eine Halstablette handelt.

5. Pharmazeutische Zusammensetzung zur Verwendung in einem Verfahren zur Behandlung einer Virusinfektion nach einem oder mehreren der vorstehenden Ansprüche, wobei die Zusammensetzung in einer Tagesdosis von etwa 2-4 mg Tyrothricin, 4-8 mg Benzalkoniumchlorid und 6-12 mg Benzocain kombiniert verabreicht wird.

## Revendications

1. Composition pharmaceutique comprenant de la tyrothricine, du chlorure de benzalkonium et de la benzocaïne destinée à être utilisée dans une méthode de traitement d'une infection virale, dans lequel l'infection virale est causée par des coronavirus.

2. Composition pharmaceutique destinée à être utilisée dans une méthode de traitement d'une infection virale selon la revendication 1, dans laquelle l'infection virale est provoquée par le coronavirus SARS-CoV-2.

3. Composition pharmaceutique destinée à être utilisée dans une méthode de traitement d'une infection virale selon la revendication 1 ou 2, comprenant environ 0,5 mg de tyrothricine, environ 1,0 mg de chlorure de benzalkonium et environ 1,5 mg de benzocaïne en dose unique et un ou plusieurs excipients pharmaceutiques acceptables.

4. Composition pharmaceutique destinée à être utilisée dans une méthode de traitement d'une infection virale selon une ou plusieurs des revendications précédentes, qui est un comprimé pour la gorge.

5. Composition pharmaceutique destinée à être utilisée dans une méthode de traitement d'une infection virale selon une ou plusieurs des revendications précédentes, dans laquelle la composition est administrée en une dose quotidienne d'environ 2 à 4 mg de tyrothricine, 4 à 8 mg de chlorure de benzalkonium et 6 -12 mg de benzocaïne combinée.
